(19) Europäisches Patentamt
European Patent Office
Office européen des brevets

(11) Publication number: **0 051 888 B1**

(12) **EUROPEAN PATENT SPECIFICATION**

(45) Date of publication of patent specification: **07.03.84**

(51) Int. Cl.³: **C 02 F 3/30**, C 02 F 3/28

(21) Application number: **81201181.5**

(22) Date of filing: **30.10.81**

(54) **Process for the purification of waste water and/or waste water sludge.**

(30) Priority: **07.11.80 NL 8006094**

(43) Date of publication of application:
**19.05.82 Bulletin 82/20**

(45) Publication of the grant of the patent:
**07.03.84 Bulletin 84/10**

(84) Designated Contracting States:
**AT BE CH DE FR GB IT LI LU NL SE**

(56) References cited:
**US - A - 4 182 675**

**CHEMICAL ABSTRACTS, vol. 87, no. 18, October 31, 1977, page 282, abstract 140611w Columbus, Ohio, US J. BISOGNI et al. "Denitrification using thiosulfate and sulfide"**
**CHEMICAL ABSTRACTS, vol. 89, no. 12, September 18, 1978, page 276, abstract 94551t Columbus, Ohio, US C.T. DRISCOLL et al. "The use of sulfur and sulfide in packed bed reactors for autotrophic denitrification"**

(73) Proprietor: **Gist - Brocades N.V.
Wateringseweg 1 P.O. Box 1
NL-2600 MA Delft (NL)**

(72) Inventor: **Mulder, Arnold
Rietdekkersdreef 710
NL-7328 AK Apeldoorn (NL)**

(74) Representative: **Van der Straaten, Jan Anthony et al,
c/o GIST-BROCADES N.V. Patents and Trademarks Department Wateringseweg 1 P.O. Box 1
NL-2600 MA Delft (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 051 888 B1**

Courier Press, Leamington Spa, England.

Process for the purification of waste water and/or waste water sludge

The invention is relating to a process for the purification of waste water and/or waste water sludge, comprising subjecting the waste water and/or the waste water sludge to methane fermentation and oxidizing the reduced compounds present in the liquid effluent of the methane fermentation by aeration.

Such a process is generally known and is applied in particular to waste water and/or waste water sludge of fermentation industries (Sew. W. J. *1948*, 1084 and *1949*, 1000, 294, 491, 700, 1028 and Ind. Eng. Chem., *1949*, 1535).

In the first stage of such a process the COD present in the waste water (sludge) is converted for the major part into methane and carbon dioxide, while hydrogen sulphide and ammonia originate from the sulphur containing compounds and nitrogen containing compounds respectively. The hydrogen sulphide turns up in part in the methane gas and must be removed therefrom before the methane gas can be used for the energy production, while the remainder is found in the liquid effluent of the methane fermentation, in the form of unsoluble precipitates and as dissolved $H_2S$ and/or $HS^-$ ions. The ammonia dissolves mainly in the liquid effluent of the methane fermentation.

Therefore the liquid effluent of the methane fermentation is not appropriate for a direct draining; it stinks and is poisonous.

In the second stage the organic compounds dissolved in the effluent are partially degraded by aeration or are converted into sludge, which is removed and for instance is recirculated to the methane fermentation, by which means the COD of that effluent is decreased significantly and the sulphides and ammonia are oxidized into sulphate ions and nitrate ions respectively. When the aerated water is drained off the so formed nitrate gives rise to problems with the eutrofication of surface water if waste water and/or waste water sludge having a high content of nitrogen containing compounds are used. Therefore the nitrate in the aerated effluent of the methane fermentation is converted under practical conditions into nitrogen in the usual way (see for instance J.A.W.W.A., 659—662 (1969)) under anaerobic conditions in the presence of the naturally occurring denitrificating microorganisms and under application of organic compounds (methanol) as electron donor (so called denitrification). The obtained nitrogen is removed.

Several difficulties are connected with such a way of operation in the case of waste water and/or waste water sludge, which contain many sulphur containing impurities, namely

1) In the aeration reactor a large amount of oxygen is necessary for the oxidation of $H_2S/HS^-$. The air, passed through this reactor to provide for the oxygen required, becomes strongly polluted with smelling compounds such as $H_2S$ and mercaptans. Therefore follow-up treatment of this air is inevitable.
2) The sulphate which is formed in the aeration step is reconverted into sulphide during the anaerobic denitrification with all the problems connected with it, such as inhibition of the denitrification at a low sulphide concentration (1 mg/l).
3) The methane gas has to be treated in a separate way for the removal of $H_2S$.
4) At varying $NO_3^-$ concentrations the COD supply into the nitrate reducing reactor forms a large problem. Notably, excessive dosage of COD results in an anaerobic condition, wherein the sulphate formed, while acting as electron donor for the oxidation of COD, is reduced to sulphide.
5) The final waste water is oxygen free and contains toxic sulphides and a possible excess of COD from the denitrification step.

Surprisingly it was found that these problems are significantly diminished or even completely avoided, when the sulphide from the effluent of the methane fermentation is used as electron donor in the denitrification and when the remaining reduced compounds from the liquid effluent of the methane fermentation are only thereafter oxidized by aeration.

In this way the sulphides are used in an appropriate way and simultaneously converted into non troublesome sulphates according to the reaction:

$$8H^+ + 8NO_3^- + 5S^{2-} \rightarrow 4N_2 + 4H_2O + 5SO_4^{2-}.$$

In the liquid effluent from the denitrification step, the ammonia from the methane fermentation is still present; this ammonia is subsequently oxidized into nitrate by aeration, whereby the sulphide cannot disturb as this was already converted into $SO_4^{2-}$ in the denitrification step. As sulphide is no longer present, the aeration needs less air and the aeration may be more simple.

It will be appreciated that the possibility of denitrification with the application of sulphur containing compounds which are in reduced state with respect to the $SO_4^{2-}$ state ($S^{2-}$, S, $S_2O_3^{2-}$, $S_4O_6^{2-}$, $SO_3^{2-}$) as electron donors and the application of *Thiobacillus denitrificans* as a microorganism, is already mentioned in Batchelor and Lawrence, "Chemistry of Wastewater Technology", chapter 24.

However the application of a liquid effluent from methane fermentation of waste water with many S containing impurities as a source of sulphides, which serve as electron donors, is not

disclosed at all and the advantages by using such an effluent cannot be derived therefrom.

The hydrogen sulphide from the gas which is formed during methane fermentation also can serve as electron donor in the denitrification and is preferably applied for this purpose. In this way the best possible use of the products obtained in the methane fermentation can be made while in addition the gas is purified in a simple way.

If the S/N-ratio, which is reached in that way, is insufficient to reduce all $NO_3^-$, unpurified waste water containing sufficient amounts of COD is introduced into the denitrification reactor in order to achieve complete reduction of the nitrate to $N_2$. If an excess of COD is added to the denitrification reactor consequent on variations of N and S concentrations in the waste water, this excess is automatically oxidized to $CO_2$ in the next aeration step, so that this COD does not give rise to decreased purification.

The effluent from the aeration step containing nitrate ions and $SO_4^{2-}$ ions already formed during denitrification, suitably is recycled in part to the denitrification step.

To achieve complete conversion into sulphate of sulphide formed in the first step, the nitrate containing effluent, which is formed during the aeration, is partially subjected to the denitrification as a nitrate containing waste water, according to the invention and whereby the remainder is drained off.

$SO_4^{2-}$ ions present in the liquid do not disturb denitrification of nitrate ions by sulphide, since sulphide is unable to reduce the $SO_4^{2-}$ ions.

In that way an integrated system is obtained, whereby the effluent of the methane fermentation of waste water and/or waste water sludge is first used as a source of electron donor for denitrification, the effluent of the denitrification is aerated under formation of nitrate, whereupon this nitrate containing liquid is recycled in part to the denitrification.

It will be appreciated that from chapter 15 of "Nitrification and Denitrification in Waste Water Treatment" an integration of a denitrification step and a nitrification step is well known in its own. However in the system disclosed therein unpurified waste water is used as a source of electron donor for the denitrification and is combined with nitrate containing waste water, which is obtained by aeration of the effluent of the denitrification.

Indications for the application of the liquid effluent from the methane fermentation containing many sulphur compounds as a source of electron donors in the denitrification cannot be derived therefrom.

According to the process of this invention, a purification of waste water and/or waste water sludge, wherein nitrogen and sulphur containing impurities are present, is achieved and, due to the methane fermentation of the COD of the waste water and/or the waste water sludge methane gas is obtained, which may serve as a source of energy. Owing to the use of the liquid effluent and the gas of the methane fermentation as source of $H_2S$, $HS^-$ and/or $S^{2-}$, which serve as electron donors for the denitrification, a reliable, economical removal of nitrogen- and reduced sulphur compounds is reached.

The total amount of nitrogen, which is removed, depends on the volume ratio between the nitrate containing effluent, which is subjected to the denitrification, and the effluent of the methane fermentation, which is directed to the denitrification.

Preferably this volume ratio is at least 1:1. In this case at least 50% of the nitrogen is removed. Most preferably the indicated ratio is 4:1 to 9:1, because the amount of nitrogen, calculated on the basis of the waste water and/or waste water sludge, which will be removed within these ratios, namely 80 to 90%, will suffice for practical purposes.

Due to the high volume ratios of 4:1 to 9:1 the flow rate of the water through the denitrification reactor and nitrification reactor increases tremendously.

Therefore one or more of these steps of this process are carried out preferably in the so called biologically fluidized bed reactor, wherein the biomass grows attached to a solid heavy carrier and thus is not leaving the reactor under high flow rate conditions.

More preferably in the denitrification such system is used. Such a biomass attached to a heavy carrier system may be prepared e.g. by a process of the EP—A—0 028 846, the relevant parts of which are incorporated herein by reference.

The invention is now further described with reference of the figures.

Figure 1 is representing the basic principle of the invention.

Figure 2 is representing schematically the preferred embodiment of the invention.

According to the Figure 1 nitrate containing waste waster is supplied by means of pipeline 1 to the denitrification reactor 2 having an upgoing liquid flow (up-flow reactor). The liquid effluent of the methane fermentation of waste water and/or waste water sludge with N and S containing impurities is pumped by means of pipeline 3 to the reactor 2. $H_2S$ containing gas, derived from the methane fermentation, is pumped by means of pipeline 4 into the bottom of an absorption column 5, wherein in the upper part the liquid effluent from the denitrification reactor 2 is supplied through pipeline 6.

The gas from which $H_2S$ is absorbed in this way, evades from the absorption column 5 via pipeline 7.

The liquid wherein the $H_2S$ is absorbed leaves the absorption column 5 via pipeline 8 and is added to the liquid effluent of the methane fermentation in pipeline 3. In the denitrification reactor 2 nitrate is reduced to nitrogen, which evades via pipeline 9.

From the top of the denitrification reactor 2 an amount of purified waste water is drained away by means of the pipeline 10. This amount of purified waste water is equal to the amount of nitrate containing waste water, which is supplied by pipeline 1 together with the amount of liquid effluent of the methane fermentation which is supplied by pipeline 3.

According to Figure 2 the waste water and/or waste water sludge, containing besides the COD nitrogen- and sulphur compounds, is supplied by means of pipeline 11 to a methane fermentation reactor 12. From this reactor ammonia and sulphide containing liquid effluent is supplied by means of pipeline 13 to a denitrification reactor 15, to which on the other hand nitrate containing waste water from the subsequent stage is added via pipeline 16 and via pipeline 17 non-purified COD containing waste water, coming from pipeline 11, are added.

A gas stream containing $CO_2$, $CH_4$ and $H_2S$ which is formed in the methane fermentation is supplied by means of pipeline 14 to a washing column 24, wherein the $H_2S$ is removed from the gas by washing with denitrified waste water which is supplied by pipeline 25.

The gas from which $H_2S$ is removed evades from the washing column 24 by means of the pipeline 26 and the washing liquid wherein $H_2S$ has been absorbed is led by means of pipeline 27 into the pipeline 17 and from there to the denitrification reactor 15. In the denitrification reactor the nitrate, which is present in the liquid mixture, is reduced to nitrogen which evades by means of pipeline 18. Denitrificated effluent which contains ammonia, originating from the effluent of the methane fermentation and sulphate, formed by the denitrification from sulphide, is led to an aerobic reactor 20 by means of pipeline 19. To this aerobic reactor 20 oxygen for the oxidation of the ammonia is supplied by means of pipeline 21.

The carbon dioxide and the remaining air arising from the oxidation of the COD evade by means of pipeline 22. Nitrificated effluent leaves the reactor 20 by means of pipeline 23. A part of this effluent is drained away as purified water, the remaining part is recirculated by means of pipeline 16 to the denitrification reactor 15.

Example I

The following experiment was carried out in an equipment according to Figure 1, of which the reactor 2 had a height of 1.1 m and a diameter of 0.09 m (volume 6.5 l).

Into reactor 2 was introduced through pipeline 1 an aqueous nitrate solution (400 mg nitrate nitrogen/l; volume 40 l/d; total amount of nitrate 1140 mmol $NO_3$/d).

40 l/d of liquid effluent from a methane fermentation reactor (sulphur content 240 mg/l) were supplied through pipeline 3, and through pipeline 4 a corresponding amount of gas formed in the methane fermentation, in which column $H_2S$ was washed out by a stream of liquid coming from a denitrification reactor through pipeline 6, was supplied to the absorption column 5. Purified gas escaped through pipeline 7. The liquid from the absorption column was mixed with liquid effluent in pipeline 3.

The liquid in the denitrification reactor was stirred with 2 rpm; in the reactor the pH was 7.3—7.4 and the temperature varied between 20 and 30°C.

The effluent from the reactor (pipeline 10) contained a negligible amount of nitrate and 400 mg $SO_4^{2-}$/l.

Per day about 7 l of gas were formed (pipeline 9) which were consisting of 80% of $N_2$ and for the remainder of $CO_2$.

The presence of $CO_2$ indicates, that the nitrification took place also by means of COD in the waste water, but the high nitrogen content of the gas and the high sulphate content of the effluent introduced by pipeline 10 point at predominant contribution of the sulphides to the denitrification.

In this experiment a considerable denitrification capacity, 1.2 kg $NO_3^-$-nitrogen/m$^3$ reactor volume per day, was achieved.

Example II

An experiment was carried out in an equipment according to Figure 2, of which the methane fermentation reactor 12 had an effective volume of 35 l, the denitrification reactor 15 had an effective volume of 6.5 l and the aerobic reactor 20, which was combined with a settler to prevent that sludge is carried away by nitrificated effluent, had an effective volume of 13 l (total effective volume of reactor 20 together with the settler was 23 l).

The methane fermentation reactor 12 was prepared for the experiment by passing through during three months waste water containing COD, nitrogen compounds and sulphur compounds in an amount of 50 l per day.

At the end of this period this reactor contained 330 g of sludge, calculated as organic material.

The denitrification reactor 15 was prepared in the following way. It was filled half way with active sludge originating from the aeration basin of the "R.W.Z.I." (municipal waste water-treatment plant) in Renkum-Wageningen, whereupon it was filled up with effluent from a methane fermentation reactor. After two days 2 l liquid was withdrawn from the reactor, and the reactor was filled up again with fresh effluent from the methane fermentation reactor. These manipulations were continued during one month, whereafter a continuous flow from the methane fermentation reactor was passed through beginning with an amount of 10 l/d. Thereafter the amount passed through was regularly increased during one month to 50 l/d (residence time, 3.1 h).

The nitrification reactor 20 was prepared by introduction of 7.5 l of liquid sludge from the aeration basin of the "R.W.Z.I." in Renkum-Wageningen. At the end of the experiment the reactor contained 50 g of sludge, calculated as dry organic material.

After all three reactors had been prepared in this manner, the experiment was started by introduction of water containing COD, nitrogen compounds and sulphur compounds, in an amount of 50 l/d through pipeline 11 in the methane fermentation reactor 12, by continuous introduction of liquid effluent of this reactor by means of pipeline 13 in the denitrification reactor 15 and by pumping the gas formed in the methane-fermentation through pipeline 14 in the gas-washer 24, whereby liquid effluent of gas-washer 24 was passed through pipeline 27 into the reactor 15, a part of the liquid effluent from the reactor 15 was passed through pipeline 25 into gas-washer 24 (in order to wash the methane gas) and the washed methane gas exited through pipeline 26, while the remaining effluent from the denitrification reactor by means of pipeline 19 was led into the nitrification reactor and the effluent of that reactor by means of pipeline 16 was brought back into the denitrification reactor. After all the reactors and pipelines had been filled in this manner, an amount of effluent of the reactor 20 was drained away through pipeline 23, corresponding with the amount of water, containing COD, nitrogen compounds and sulphur compounds which had been introduced through pipeline 11.

After the installation had been working in this manner during one month, a stable situation I, was established.

The conditions prevailing in each reactor reached after establishment of the stable situation I, are indicated in Table A, the composition of the waste water containing COD, nitrogen compounds and sulphur compounds and the composition of the various effluents are shown in Table B and the amounts of gas developed in the methane fermentation and in the denitrification as well as the composition of those gas-streams are shown in Table C.

Subsequently, the COD, nitrogen compounds and sulphur compounds containing waste water, which had been introduced through pipeline 11, was raised to 51 l/d, while the composition of this waste water changed too, and the other flows were adjusted. While passing through pipeline 16, the recirculation stream was also supplied with a volume of a sodium nitrate solution (not indicated in Figure 2), in order to prevent recirculation of a too large amount of effluent from reactor 20.

After one month a stable situation II was established. The conditions and the composition of the various streams as well as the amounts of gas and their compositions in this experiment are shown in the Tables A, B, and C.

TABLE A
Process conditions

| | Reactor 12 | | Reactor 15 | | Reactor 20 | |
|---|---|---|---|---|---|---|
| | I | II | I | II | I | II |
| effective vol. (l) | 35 | 35 | 6.5 | 6.5 | 13 | 13 |
| amount of sludge (g. org. material) | 330 | 330 | 66 | 66 | 50 | 50 |
| mean temp. (°C) | 37 | 37 | 25.5 | 25.5 | 23 | 23 |
| pH | 7.2 | 7.2 | 8.2 | 8.2 | 7.8 | 7.8 |
| recirc. through pipeline 16 (l/d) | | | 42 | 63.5 | 42 | 63.5 |
| residence time (h) | 16.8 | 16.5 | 3.1 | 3.1 | 6.2 | 6.1 |

TABLE B
Composition of liquid streams

| In pipeline | 11 | | 13 | | 19 | | 16/23 | |
|---|---|---|---|---|---|---|---|---|
| situation | I | II | I | II | I | II | I | II |
| $SO_4$ mg/l | 586 | 1374 | 1 | 2 | 293 | 608 | 285 | 628 |
| sulphide (mg/l as S) | — | — | — | 378 | — | — | — | — |
| COD mg/l | 3510 | 6700 | 835 | — | — | — | 545 | 1115 |
| $NH_4^+$ (mg/l as N) | 272 | — | 403 | 295 | — | 141 | 159 | 51 |
| nitrite (mg/l as N) | — | — | — | — | — | 0 | 30.2 | 41.1 |
| nitrate (mg/l as N) | — | — | — | | — | — | 46 | 50 |
| added nitrate in pipeline 16 (g/d as $NO_3^-$) | | | | | | | | 11 |

TABLE C
Amount of gas formed and gas composition

| Situation | in pipeline 14* I | in pipeline 14* II | in pipeline 18 I | in pipeline 18 II |
|---|---|---|---|---|
| amount of gas measured (l/d, at stand. temp. and pressure) | 55 | 102.6 | 2.98 | 7.14 |
| $N_2$ | 2.9 | 1.5 | 85.9 | 85.2 |
| $O_2$ | 0.34 | — | 1.9 | — |
| $CH_4$ | 73.4 | 67.7 | 8.6 | 7.9 |
| $CO_2$ | 19.9 | 26.3 | 3.2 | 4.3 |

*): after removal of $H_2S$.

These results show, that with the working-method of the present invention an efficient denitrification can be achieved under variable conditions without the occurrence of problems related to the presence of sulphur compounds in the waste water to be treated.

It follows from the figures of the amounts of sulphur compounds in the various streams that in these experiments losses of sulphur have appeared.

This may be due to various reasons.

a) some sulphide is lost with the gas of the methane fermentation.
b) during the process some FeS is formed, which is not expressed in the sulphur balance.
c) in places where oxygen is present (tube connections, head of the reactor), formation of elementary sulphur takes place, which also does not appear in the sulphur balance.

Example III

The following experiment was carried out in an equipment according to Figure 1. In this particular experiment however there was no $H_2S$-stripping of the biogas provided. This means that the gasflow through the pipelines 4 and 7 was 0. The recirculation of water through the pipelines 6 and 8 was however maintained at 600 l/h. The reactor 2 had a height of 12 m and a diameter of 0.2 m and had a volume of 400 l. This reactor was loaded at the start of the experiment with 100kg of sand of 0.8—1.2 mm particle diameter. This sand was maintained in the fluidized state with sufficient recirculation waterflow through pipelines 6 and 8 mentioned above. A nitrite containing solution (333 g $NaNO_3$/kg) was fed in the reactor 2 through pipeline 1 at a rate of 1 l/h. Through pipeline 3 350 l/hr of anaerobic, sulfide containing stinking waste water (originating from a waste water methane reactor) was fed into reactor 2. The sulfide concentration in this waste water was about 150 mg $S^{2-}$/l. The pH of reactor 2 was maintained at 7.6—7.8 and the temperature was maintained at 35°C. The biomass concentration in the biomass coated sand fluidized bed in reactor 2 was about 35—40 g of volatile suspended solids per kg.

The purified, odourless waste water flowing through pipeline 10 contained above 450 mg $SO_4^{2-}$/l. The gas production through pipeline 9 averaged about 1200 l/d and the composition of the gas was 65% $N_2$, 20% $CH_4$ and 15% $CO_2$. The denitrification capacity can thus be estimated at 2.5 kg $N_2/m^3$ reactor volume/d.

**Claims**

1. Process for the purification of waste water and/or waste water sludge, wherein the waste water and/or the waste water sludge is/are subjected to a methane fermentation and the reduced compounds present in the liquid effluent of the methane fermentation are oxidized by aeration, characterized in that the sulphides in the effluent of the methane fermentation are used as electron donors in a subsequent denitrification step and only thereafter the remaining reduced compounds from the liquid effluent of the methane fermentation are oxidized by aeration.

2. Process according to claim 1, characterized in that $H_2S$ contained in the gas formed in the methane fermentation is also used as electron donor in the denitrification step.

3. Process according to claims 1 or 2, characterized in that a nitrate containing effluent formed during aeration is in part recycled to the denitrification step and the remaining part is drained off.

4. Process according to claim 3, characterized in that the volume ratio of the nitrate containing effluent, which is subjected to denitrification, and the effluent of the methane fermentation, which is recycled to denitrification, is at least 1:1.

5. Process according to claim 4, characterized in that the volume ratio is 4:1 to 9:1.

6. Process according to claim 5, characterized in that the denitrification is carried out in a reactor wherein activated sludge is attached to a solid heavy carrier which does not leave the reactor under high flow rate conditions.

## Revendications

1. Procédé pour la purification d'eau usée et/ou de boue résiduaire, dans lequel l'eau usée et/ou la boue résiduaire est/sont soumis(es) à une fermentation méthanique et les composés réduits en présence dans l'effluent liquide de la fermentation méthanique sont oxydés par aération, caractérisé en ce que les sulfures de l'effluent de la fermentation méthanique sont utilisés comme donneurs d'électrons à un stade de dénitrification ultérieur et les composés réduits restants de l'effluent liquide de la fermentation méthanique ne sont oxydés qu'ensuite par aération.

2. Procédé suivant la revendication 1, caractérisé en ce que le $H_2S$ contenu dans le gaz formé pendant la fermentation méthanique est également utilisé comme donneur d'électrons au stade de dénitrification.

3. Procédé suivant la revendication 1 ou 2, caractérisé en ce qu'un effluent contenant du nitrate formé pendant l'aération est recyclé pour partie au stade de dénitrification et la partie restante est rejetée.

4. Procédé suivant la revendication 3, caractérisé en ce que le rapport volumique de l'effluent contenant du nitrate, qui est soumis à la dénitrification, à l'effluent de la fermentation méthanique, qui est recyclé à la dénitrification, est d'au moins 1:1.

5. Procédé suivant la revendication 4, caractérisé en ce que le rapport volumique est de 4:1 à 9:1.

6. Procédé suivant la revendication 5, caractérisé en ce que la dénitrification est exécutée dans un réacteur dans lequel la boue activée est fixée sur un support solide pesant qui ne quitte pas le réacteur dans des conditions d'écoulement rapide.

## Patentansprüche

1. Verfahren zur Reinigung von Abwasser und/oder Abwasserschlamm, wobei das Abwasser und/oder der Abwasserschlamm einer Methanfermentation unterworfen wird/werden und die in dem flüssigen Ausfluss der Methanfermentation vorhandenen reduzierten Verbindungen durch Belüftung oxidiert werden, dadurch gekennzeichnet, dass die Sulfide in dem Ausfluss der Methanfermentation in einer nachfolgenden Denitrifikationsstufe als Elektronendonatoren verwendet werden und die verbleibenden reduzierten Verbindungen aus dem flüssigen Ausfluss der Methanfermentation erst danach durch Belüftung oxidiert werden.

2. Verfahren nach Anspruch 1, dadurch gekennzeichnet, dass in dem bei der Methanfermentation gebildeten Gas enthaltenes $H_2S$ in der Denitrifikationsstufe ebenfalls als Elektronendonator verwendet wird.

3. Verfahren nach Anspruch 1 oder 2, dadurch gekennzeichnet, dass ein während der Beluftung gebildeter, Nitrat enthaltender Ausfluss zum Teil in die Denitrifikationsstufe zurückgeführt wird und der verbleibende Teil abgeführt wird.

4. Verfahren nach Anspruch 3, dadurch gekennzeichnet, dass das Volumenverhältnis des Nitrat enthaltenden Ausflusses, der der Denitrifikation unterworfen wird, und des Ausflusses der Methanfermentation, der in die Denitrifikation zurückgeführt wird, mindestens 1:1 beträgt.

5. Verfahren nach Anspruch 4, dadurch gekennzeichnet, dass das Volumenverhältnis 4:1 bis 9:1 beträgt.

6. Verfahren nach Anspruch 5, dadurch gekennzeichnet, dass die Denitrifikation in einem Reaktor ausgeführt wird, in dem Belebtschlamm an einen festen schweren Träger gebunden wird, der den Reaktor unter den Bedingungen einer hohen Strömungsgeschwindigkeit nicht verlässt.

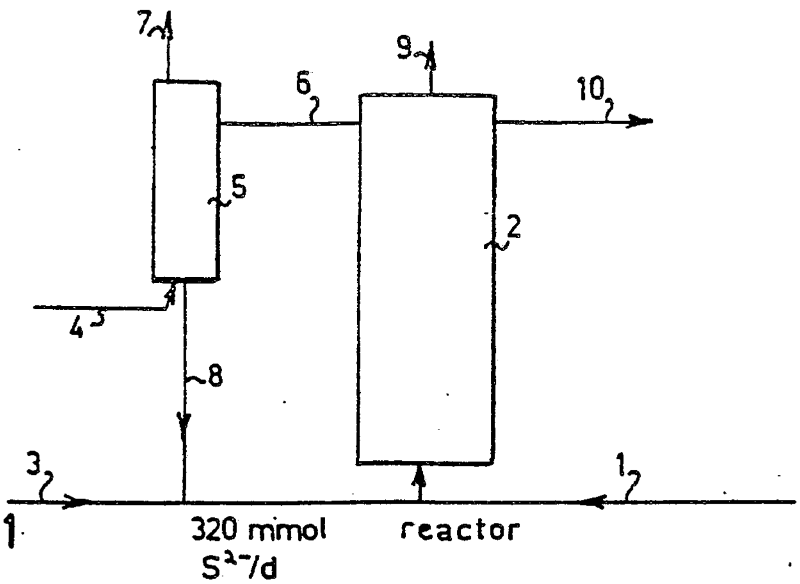
7
9
6
10
5
2
4
8
3
1
320 mmol
S2-/d
reactor
FIG.1

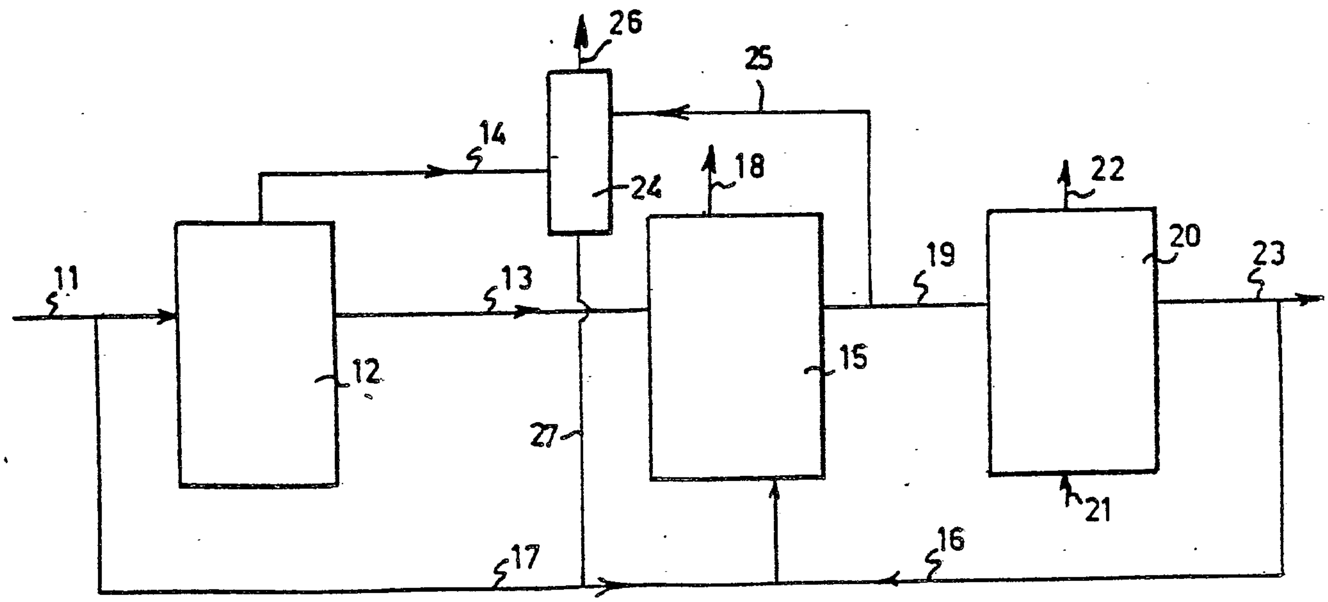
26
25
14
18
22
24
11
13
19
20
23
12
15
27
21
17
16
FIG.2